# EUROPEAN PATENT SPECIFICATION

(11) **EP 2 842 487 B1**
(45) Date of publication and mention of the grant of the patent: **26.06.2019**
(21) Application number: 14185045.3
(22) Date of filing: 08.04.2010
(51) Int. Cl.: A61B 5/06

(54) **Locatable catheter**
Lokalisierbarer Katheter
Cathéter localisable

(30) Priority: 08.04.2009 US 167839 P
(43) Date of publication of application: 04.03.2015
(62) Divisional of application: 10159373.9
(73) Proprietor: Covidien LP, Mansfield, MA 02048 (US)
(72) Inventor: Greenburg, Benny, 45100 Hod HaSharon (IL); Averbuch, Dorian, 4732157 Ramat Hasharon (IL); Zur, Oded, 44862 Kochav-Yaír Zur Yigal (IL)
(74) Representative: Maschio, Antonio

(56) References cited:
- WO-A1-00/10456
- WO-A1-96/05768
- WO-A1-97/00043
- WO-A1-97/29684
- WO-A1-99/18852
- WO-A2-03/086498
- US-A1- 2002 062 203

## Description

### FIELD OF THE INVENTION

The field of the present invention pertains generally to the navigation of a device, such as a catheter, through a branched structure and the monitoring of a position of said device once a target location has been reached. More specifically, the present invention relates to a locatable catheter for navigating through body lumens and providing a delivery conduit for other devices.

### BACKGROUND OF THE INVENTION

WO 99/18852 discloses a directable catheter which comprises a flexible elongate tube having a distal end, a proximal end, an inner surface and an outer surface. The distal end has a tip that comprises three coils and, extending from the coils leads that are electrically connected with an instrument capable of electrical communication with the coils. The coils are applied to the tip of the catheter. Although the coils may be placed end-to-end along the longitudinal axis of tube, it is preferred that the coils are layered as inner, central and outer coils. It is preferred that the coils are applied to outer surface of the catheter. Other configurations are possible, however this configuration is generally preferred because it allows the catheter core to remain free for the delivery of materials for diagnostic or interventional actions by the operating physician. Although the coils may be placed end-to-end along the longitudinal axis of tube, it is preferred that the coils are layered as inner, central and outer coils. An alternative catheter comprises an elongate tube having a tip on which is placed three coils and the tip of the catheter is covered with sheath.

The most common interventional procedure in the field of Pulmonary Medicine (i.e., medicine pertaining to the respiratory system) is bronchoscopy, in which a bronchoscope is non-invasively inserted into the airways through the patient's nose or mouth. Bronchoscopy is generally a non-life threatening procedure. The structure of a bronchoscope generally includes a long, thin, flexible tube that typically contains three elements: an illumination assembly for illuminating the region distal to the bronchoscope's tip via an optical fiber connected to an external light source, an imaging assembly for delivering back a video image from the bronchoscope's distal tip, and a lumen or working channel through which instruments may be inserted, including but not limited to diagnostic (e.g., biopsy tools) and therapeutic (e. g.
laser, cryo or RF tissue elimination probes) instruments. The distal tip of a bronchoscope is steerable and therefore rotating a lever placed at the handle of the bronchoscope actuates the steering mechanism by deflecting the tip in two opposite directions. Bronchoscopes are limited, however, in how far they may be advanced through the airways due to their size. Typically, a bronchoscope is much wider than other types of catheters, mainly due to the size constraints placed on their design by the camera. Unfortunately, the lesion or target of interest is often located deeper in the lungs than a bronchoscope can travel. Hence, three-dimensional location technology has been developed that allow the navigation of a steerable catheter deep into the lungs. The catheter includes a sensor that can be detected magnetically with great precision. Of particular relevance to the present invention are the devices and methods described in the following references: PCT Patent Publication No. WO 03/086498 entitled "Endoscope Structure and Techniques for Navigation in a Branched Structure" to Gilboa; 7,233,820 entitled "Endoscope Structures And Techniques For Navigating To A Target In Branched Structure" to Gilboa; 6,947,788 entitled "Navigable Catheter" to Gilboa; 6,833,814 entitled "Intrabody Navigation System For Medical Applications" to Gilboa et al.; 6,711,429 entitled "System And Method For Determining The Location Of A Catheter During An Intra-Body Medical Procedure" to Gilboa et al.; 6,615,155 entitled "Object Tracking Using A Single Sensor Or A Pair Of Sensors" to Gilboa; 6,593,884 entitled "Intrabody Navigation System For Medical Applications" to Gilboa et al.; 6,380,732 entitled "Six-Degree Of Freedom Tracking System Having A Passive Transponder On The Object Being Tracked" to Gilboa; 6,188,355 entitled "Wireless Six-Degree-Of-Freedom Locator" to Gilboa.

These references describe methods and devices in which locatable guides ("LGs"), enveloped by sheaths, are used to navigate to a location within the lung. The guide/sheath combination is inserted into the lung via the working channel of a bronchoscope. Once the tip of the guide is located at its target, a lock, which is placed at the orifice ("connection port") of the bronchoscope's working channel, is operated to prevent the sheath from sliding in or out of the bronchoscope. The guide is then withdrawn from the sheath, leaving the sheath in place to guide a tool to the required target location.

Once the target has been reached with the LG, the LG is removed, leaving the sheath in place as a conduit to the target for other tools. However, the present design includes most or all of the location technology on the LG.

Hence, once the LG is removed from the sheath, the physician is assuming that the sheath remains in close proximity to, and pointed at, the target. This may not be true as the sheath may be moved in relation to the target, e.g. due to body movements. Hence, there is a need to provide an advantageous device, method and/or system to overcome these issues.

### OBJECTS AND SUMMARY OF THE INVENTION

The present invention is disclosed in the appended set of claims. The present invention addresses the identified needs by providing a locatable sheath that can be used in conjunction with, or instead of, an LG. Monitoring of a position of a device is advantageously provideable, for instance once a target location has been reached.
Adding a sensor to a sheath may add the complication of maintaining an open lumen through the sheath. As such, various sensor designs are provided in embodiments that do not interfere with the sheath lumen.

Accordingly, embodiments of the present invention preferably seek to mitigate, alleviate or eliminate one or more deficiencies, disadvantages or issues in the art, such as the above-identified, singly or in any combination.

One aspect of the present invention provides a locatable medical instrument insertable through a working channel of a bronchoscope, comprising: a sheath having at least three coils embedded therein, the at least three within each coil of the at least three coils, and each of the at least three coils being within a wall defining a longitudinal lumen extending through the sheath, wherein each of the at least three coils is configured to generate a signal when placed in an electromagnetic field, the signal indicative of a strength of the electromagnetic field and the position of the sheath, and wherein a first coil defines a first plane that is perpendicular to a longitudinal axis of the sheath and said medical instrument being characterised in that the second and third coils define a second plane and a third plane which are perpendicular to each other and angled relative to the first plane and the longitudinal axis of the sheath.

The sensor is comprised of three coils of wire, each at an angle to the others but sharing a common center point, in order to reduce the overall longitudinal dimension of the sensor. By reducing the longitudinal dimension, the impact the sensor has on the flexibility of the distal tip is minimized.

### BRIEF DESCRIPTION OF THE DRAWINGS

Figure 1 is a perspective view of locatable guide within a sheath; and,
Figure 2 is a perspective view of an embodiment of the present invention.

### DESCRIPTION OF EMBODIMENTS

Specific embodiments of the invention will now be
described with reference to the accompanying drawings. This invention may, however, be embodied in many different forms and should not be construed as limited to the embodiments set forth herein; rather, these embodiments are provided so that this disclosure will be thorough and
complete, and will fully convey the scope of the invention as defined by the appended patent claims to those skilled in the art. The terminology used in the detailed description of the embodiments illustrated in the accompanying drawings is not intended to be limiting of the invention. In the drawings, like numbers refer to like elements.

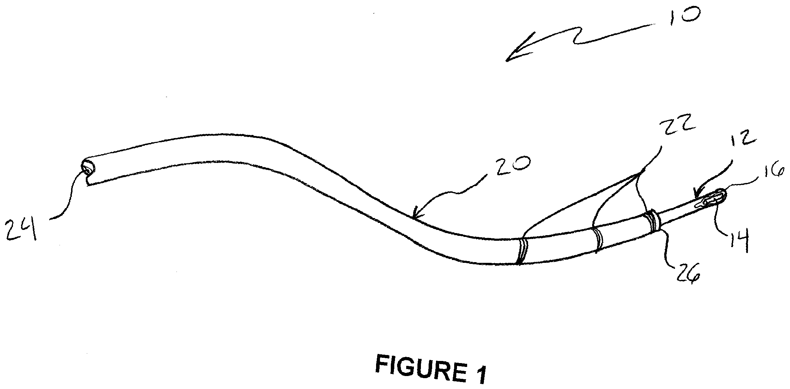

Turning now to the Figures and first to Figure 1, there is shown a device 10.Device 10 generally comprises a locatable guide ("LG") 12 and a locatable sheath 20. It is understood that locatable sheath 20 could be used with or without LG 12. The LG 12 includes a sensor 14 embedded in a distal tip 16 thereof. The distal tip 16 is shown with a cutout in order to view the embedded sensor 14. Examples of LG 12 embodiments are shown and described in PCT Patent Publication No. WO 03/086498 entitled "Endoscope Structure and Techniques for Navigation in Branched Structure" to Gilboa.

These LG 12 embodiments are steerable probes that incorporate six degree-of-freedom ("6 DOF") sensors that operate by sensing externally-generated magnetic fields. For example, three magnetic fields may be generated, each using a separate frequency. The three magnetic fields vary in intensity throughout an operational area. Placing three orthogonally-oriented coils in the operational area results in signals generated by the coils that indicate three-dimensional location and three-dimensional orientation of the sensor 14. Examples of such sensors are shown in Figures 1-3 of U.S. Patent 6,947,788, entitled "Navigable Catheter." Specific descriptions of the embodiments shown in these figures are found in the specification of this patent beginning at column 10 line 7 through column 11, line 24 and column 12 line 32 through column 13 line 5. These particular passages of U.S. Patent 6,947,788 are referenced.

The sheath 20 includes one or more coils 22 that are either embedded in its wall or are attached to an outside surface thereof. The sheath 20 also defines a central lumen 24 that is unobstructed by the coils 22. One embodiment of the sheath 20 includes only a single coil 22 proximate its distal tip 26. The coil 22 is used to generate signals indicating its location in a variety of ways.

For example, the single coil can generate signals using the same magnetic fields utilized by the LG 12. Hence, after the LG 12 is removed, the single coil 22 may be used to generate a signal indicating its three-dimensional location in the operational field.

Alternatively, a field may be generated on a fourth frequency such that the sensor 22 may be used concurrently with the sensor 14 of the LG 12. If the sensor 22 is dedicated to a single field on a fourth frequency, the sensor would not be useful for three-dimensional position data but could be used to generate a signal indicating that it has moved out of position after the LG 12 is withdrawn.

Another device, shown in Figure 1, utilizes multiple coils 22. The coils 22 are spaced apart along the length of the sheath 20. The coils 22 provide position data of various points along the length of the sheath 20, thereby "painting a picture" of the sheath. As such, even though the coils 22 are not providing 6 DOF data, when viewed together they provide an indication of the location and orientation of the sheath 20.Hence, this embodiment of sheath 20 may be used to both navigate to the target and provide a conduit for tools, possibly obviating the need for the LG 12.

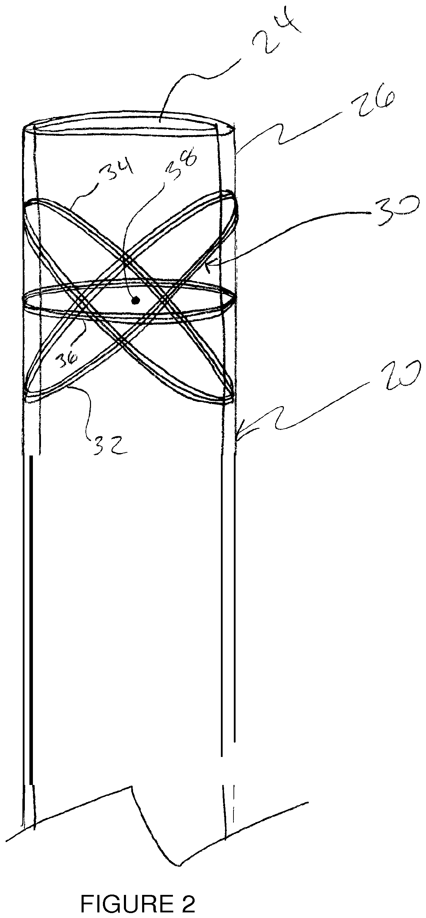

Referring now to Figure 2, there is shown an embodiment of a sheath 20 with a 6 DOF sensor 30 incorporated into its distal tip 26. The sheath 20 is shown as transparent such that the details of the sensor 30 may be more easily displayed. The sensor 30 includes three coils 32, 34 and 36. Coils 32 and 34 are angled, while coil 36 is relatively circumferential to the sheath 20. In the illustrated embodiment coil 36 defines a plane that is approximately perpendicular to a longitudinal axis of said sheath. Coils 32 and 34 lie in planes that are relatively perpendicular to each other. All three coils share a common center point 38. By sharing a common center point 38, the longitudinal dimension of the overall sensor is minimized, thereby reducing the impact of the sensor 30 on the flexibility of the sheath 20. This design represents an advantage over prior art designs, such as that shown and described in International Publication Number WO 97/29684, to Acker. Acker discloses several coil designs that leave a central lumen open but are longitudinally extended, resulting in a reduced flexibility of the catheter in order to maintain a constant spatial relationship between the coils.

As stated above, it is to be understood that the sheath designs herein are to be used with an electromagnetic navigation system, with or without a locatable guide, for example, a system for navigating through a
branched structure that includes a sheath 20, such as that shown in Figure 1 or 2, having a distal end 26 and defining a lumen 24 extending longitudinally therethrough.

The sheath 20 includes a first electromagnetic field sensor comprising at least one coil 22. The device of Figure 1 shows a sensor with multiple coils 22 while the embodiment of Figure 2 shows an embodiment with a single coil 22. The system, as discussed in reference to Figure 1, includes a probe (also referred herein as a "Locatable Guide" or **"LG"**) 12, sized to extend through the sheath 20. The LG 12 has a second electromagnetic field sensor 14 embedded in a distal tip thereof. Though the second electromagnetic field sensor 14 is preferably a **six** degree-of-freedom sensor, such as that shown in U.S. Patent 6,947,788, referred to above, other sensor designs are acceptable. Particular sensor embodiments from U.S. Patent 6, 947, 788 are cited above referenced.

The system also includes an electromagnetic field generation device, capable of creating electromagnetic fields having a plurality of frequencies and able to induce signals in sensors such as those used as first and second sensors. An example of such an electromagnetic field generation device is shown in U.S. Patent Publication No. 20090284255, to Zur, and entitled, "Magnetic Interference Detection System and Method." More specifically, Figure 6 of this reference shows a stacked coil generator, which is described in paragraphs [0025] through [0029].

A method, which is not claimed, for accessing a target in a branched structure comprises the following steps: providing
an electromagnetic field generator defining a sensing volume containing said branched structure; advancing a probe through said branched structure, said probe including an electromagnetic field sensor capable of sensing electromagnetic fields generated by said generator and creating locations signals as a result; using said locations signals to locate said target; advancing a sheath over said probe to said target, said sheath having an electromagnetic field sensor at a distal end thereof that is capable of sensing electromagnetic fields generated by said generator and creating locations signals as a result; removing said probe proximally from said sheath; monitoring a location of said sheath using said locations signals, to ensure the distal end of said sheath remains at said target; and accessing said target with one or more instruments by passing said one or more instruments through said sheath.

Monitoring a location of said sheath may comprise reading signals from said sensor on said sheath at a frequency that is independent of frequencies of signals from said sensor on said probe.

Alternatively, or in addition, monitoring a location of said sheath may comprise reading signals from said sensor on said sheath at a frequency that is the same as frequencies of signals from said sensor on said probe.

Preferably, the method makes use of devices and systems as described above with reference to various embodiments thereof.

In some embodiments the branched structure may be the respiratory system. Accessing the target structure may be bronchoscopy. In such embodiments, the method does not comprise surgical steps and is performed non-invasively in a non-life threatening manner. Navigation in the urinary tract is another example. The improved navigation provided by embodiments of the invention facilitates performance of such procedures.

In other embodiments, the branched structure may be a different branched tubular structure having a plurality of branches and bifurcations, such as a natural structure e.g. present in certain plants, or a man made structure such as certain pipe systems.

In other examples, which may not be covered by embodiments of the method, some embodiments of the device and/or system may be applied in the human portal vein tree, or other blood vessel branched structures.

Although the invention has been described in terms of particular embodiments and applications, it should be appreciated that other embodiments and applications also fall within the scope of the present invention as defined by the appended patent claims. One of ordinary skill in the art, in light of the teaching, can generate embodiments and modifications without departing from the scope of the claimed invention.

For example, each of the embodiments is described as having sensors incorporated into a sheath 20 that are passive. In other words, the described sensors generate signals when they pass through magnetic fields or reside within an electromagnetic field. However, one skilled in the art will understand that any or all of the coils of the sensors could be actively energized to generate magnetic fields. The generated magnetic fields could then be detected by external passive sensors and used to calculate a position and/or orientation of the coil(s). Accordingly, it is to be understood that the drawings and descriptions herein are proffered by way of example to facilitate the comprehension of the invention and should not be construed to limit the scope thereof as defined by the appended patent claims.

## Claims

1. A locatable medical instrument insertable through a working channel of a bronchoscope, comprising:
a sheath (20) having three coils (32, 34, 36) embedded therein, the three coils having a common center point centrally defined within each coil of the three coils, and each of the three coils being within a wall defining a longitudinal lumen extending through the sheath,
wherein each of the three coils (32, 34, 36) is configured to generate a signal when placed in an electromagnetic field, the signal indicative of a strength of the electromagnetic field and the position of the sheath (20), and said locatable medical instrument being **characterized in that** a first coil (36) defines a first plane that is perpendicular to a longitudinal axis of the sheath and said medical instrument being **characterised in that** the second and third coils (32, 34) define a second and a third plane which are perpendicular to each other and angled relative to the first plane and the longitudinal axis of the sheath.

2. The locatable medical instrument of claim 1, wherein the sheath (20) includes a generally elongated configuration and is flexible.

3. The locatable medical instrument of claim 1, wherein the sheath (20) includes a distal end (26) configured to receive a probe therethrough for positioning the sheath adjacent a target tissue.

4. The locatable medical instrument of claim 3, wherein the three coils (32, 34, 36) are positioned proximate the distal end of the sheath.

5. A system for navigating through a branched structure, comprising:
the locatable medical instrument of any of claims 1-4,
a first electromagnetic field sensor;
a probe (12) extendable through the lumen of the sheath (20) and having a second electromagnetic field sensor (14) embedded within a distal tip thereof;
an electromagnetic field generation device configured to create electromagnetic fields having a plurality of frequencies and configured to induce signals in the first and second electromagnetic field sensors,
wherein the first electromagnetic field sensor is configured to generate signals indicating its position relative to the branched structure.

6. The system of claim 5, wherein the first electromagnetic field sensor comprises a six degree-of-freedom sensor.

7. The system of claim 5, wherein the second electromagnetic field sensor (14) comprises a six degree-of-freedom sensor.

8. The system of claim 5, wherein the electromagnetic field generation device creates electromagnetic fields of at least four frequencies, three of the at least four frequencies detected by the second electromagnetic field sensor, thereby allowing the second electromagnetic field sensor to generate six degree-of-freedom location information.

9. The system of claim 8, wherein one of the at least four frequencies is detected by the first electromagnetic field sensor, thereby allowing the first electromagnetic field sensor to generate a signal including location information.

## Patentansprüche

1. Lokalisierbares medizinisches Instrument, das durch einen Arbeitskanal eines Bronchoskops eingeführt werden kann, umfassend:
eine Hülse (20), die drei Spulen (32, 34, 36) aufweist, die in ihr eingebettet sind, wobei die drei Spulen einen gemeinsamen Mittelpunkt haben, der zentral innerhalb jeder Spule der drei Spulen definiert ist, und jede der drei Spulen innerhalb einer Wand liegt, welche ein Längslumen definiert, das sich durch die Hülse erstreckt,
wobei jede der drei Spulen (32, 34, 36) ausgelegt ist, um ein Signal zu erzeugen, wenn sie in ein elektromagnetisches Feld gesetzt wird, wobei das Signal die Stärke des elektromagnetischen Felds und die Position der Hülse (20) angibt, und
wobei das lokalisierbare medizinische Instrument **dadurch gekennzeichnet ist, dass** eine erste Spule (36) eine erste Ebene definiert, die senkrecht zu einer Längsachse der Hülse ist, und wobei das medizinische Instrument **dadurch gekennzeichnet ist, dass** die zweite und dritte Spule (32, 34) eine zweite und eine dritte Ebene definieren, die senkrecht zueinander sind und in einem Winkel zur ersten Ebene und der Längsachse der Hülse stehen.

2. Lokalisierbares medizinisches Instrument nach Anspruch 1, wobei die Hülse (20) eine allgemein längliche Konfiguration enthält und flexibel ist.

3. Lokalisierbares medizinisches Instrument nach Anspruch 1, wobei die Hülse (20) ein distales Ende (26) enthält, das ausgelegt ist, um eine dort hindurchgehende Sonde aufzunehmen, um die Hülse an ein Zielgewebe anstoßend zu positionieren.

4. Lokalisierbares medizinisches Instrument nach Anspruch 3, wobei die drei Spulen (32, 34, 36) nahe dem distalen Ende der Hülse positioniert sind.

5. System zum Navigieren durch eine verzweigte Struktur, umfassend:
das lokalisierbare medizinische Instrument nach einem der Ansprüche 1 - 4,
einen ersten elektromagnetischen Feldsensor;
eine Sonde (12), die durch das Lumen der Hülse (20) ausziehbar ist und einen zweiten elektromagnetischen Feldsensor (14) aufweist, der im Inneren einer distalen Spitze derselben eingebettet ist;
eine Vorrichtung zur Erzeugung elektromagnetischer Felder, die ausgelegt ist, um ein elektromagnetisches Feld mit einer Vielzahl von Frequenzen zu erzeugen, und ausgelegt ist, um Signale in dem ersten und zweiten elektromagnetischen Feldsensor zu veranlassen,
wobei der erste elektromagnetische Feldsensor ausgelegt ist, um Signale zu erzeugen, die seine Position in Bezug auf die verzweigte Struktur anzeigen.

6. System nach Anspruch 5, wobei der erste elektromagnetische Feldsensor einen Sensor mit sechs Freiheitsgraden umfasst.

7. System nach Anspruch 5, wobei der zweite elektromagnetische Feldsensor (14) einen Sensor mit sechs Freiheitsgraden umfasst.

8. System nach Anspruch 5, wobei die Vorrichtung zur Erzeugung von elektromagnetischen Feldern elektromagnetische Felder mit mindestens vier Frequenzen erzeugt, wobei drei der mindestens vier Frequenzen von dem zweiten elektromagnetischen Feldsensor festgestellt werden, sodass der zweite elektromagnetische Feldsensor Sechs-Grad-Freiheits-Standortinformation erzeugen kann.

9. System nach Anspruch 8, wobei eine der mindestens vier Frequenzen von dem ersten elektromagnetischen Feldsensor festgestellt wird, sodass der erste elektromagnetische Feldsensor ein Signal erzeugen kann, das Standortinformation enthält.

## Revendications

1. Instrument médical localisable insérable à travers un canal de travail d'un bronchoscope, comprenant :
une gaine (20) comportant trois bobines (32, 34, 36) intégrées dedans, les trois bobines ayant un point central commun défini centralement au sein de chaque bobine des trois bobines, et chacune des trois bobines étant au sein d'une paroi définissant une lumière longitudinale s'étendant à travers la gaine,
dans lequel chacune des trois bobines (32, 34, 36) est configurée pour générer un signal lorsqu'elle est placée dans un champ électromagnétique, ledit signal étant indicatif d'une intensité du champ électromagnétique et de la position de la gaine (20), et ledit instrument médical localisable étant **caractérisé en ce que**
une première bobine (36) définit un premier plan qui est perpendiculaire à un axe longitudinal de la gaine et ledit instrument médical étant **caractérisé en ce que** les deuxième et troisième bobines (32, 34) définissent un deuxième et un troisième plan qui sont perpendiculaires l'un à l'autre et inclinés par rapport au premier plan et à un axe longitudinal de la gaine.

2. Instrument médical localisable selon la revendication 1, dans lequel la gaine (20) inclut une configuration généralement allongée et est souple.

3. Instrument médical localisable selon la revendication 1, dans lequel la gaine (20) inclut une extrémité distale (26) configurée pour recevoir une sonde à travers celle-ci pour positionner la gaine adjacente à un tissu cible.

4. Instrument médical localisable selon la revendication 3, dans lequel les trois bobines (32, 34, 36) sont positionnées à proximité de l'extrémité distale de la gaine.

5. Système de navigation à travers une structure ramifiée, comprenant :
l'instrument médical localisable selon l'une quelconque des revendications 1 à 4,
un premier capteur de champ électromagnétique ;
une sonde (12) pouvant s'étendre à travers la lumière de la gaine (20) et ayant un second capteur de champ électromagnétique (14) intégré au sein d'un bout distal de celle-ci;
un dispositif de génération de champ électromagnétique configuré pour créer des champs électromagnétiques ayant une pluralité de fréquences et configuré pour induire des signaux dans les premier et second capteurs de champ électromagnétique,
dans lequel le premier capteur de champ électromagnétique est configuré pour générer des signaux indiquant sa position par rapport à la structure ramifiée.

6. Système selon la revendication 5, dans lequel le premier capteur de champ électromagnétique comprend un capteur à six degrés de liberté.

7. Système selon la revendication 5, dans lequel le second capteur de champ électromagnétique (14) comprend un capteur à six degrés de liberté.

8. Système selon la revendication 5, dans lequel le dispositif de génération de champs électromagnétiques crée des champs électromagnétiques d'au moins quatre fréquences, trois des au moins quatre fréquences étant détectées par le second capteur de champ électromagnétique, permettant ainsi au second capteur de champ électromagnétique de générer des informations d'emplacement à six degrés de liberté.

9. Système selon la revendication 8, dans lequel l'une des au moins quatre fréquences est détectée par le premier capteur de champ électromagnétique, permettant ainsi au premier capteur de champ électromagnétique de générer un signal incluant des informations de localisation.
